# EUROPÄISCHE PATENTANMELDUNG

(11) **EP 3 042 604 A1**
(43) Veröffentlichungstag der Anmeldung: **13.07.2016**
(21) Anmeldenummer: 15400046.7
(22) Anmeldetag: 19.10.2015
(51) Int. Cl.: A61B 5/02, A61B 5/04, A61B 5/00, H04B 15/00, H02J 50/10

(54) **VERFAHREN UND VORRICHTUNG ZUR ERMITTLUNG UND AUSWERTUNG VON MESSDATEN**

(30) Priorität: 07.01.2015 DE 102015000125
(71) Anmelder: seca ag, 4153 Reinach BL 1 (CH)
(72) Erfinder: Leuner, Rüdiger, 21107 Hamburg (DE); Schmidt, Jan, 22941 Bargteheide (DE); Wjatscheslaw, Galjan, 24568 Nützen (DE)
(74) Vertreter: Patentanwälte Klickow & Partner Partnerschaftsgesellschaft mbB

(57) **Zusammenfassung**

Die Erfindung betrifft eine Vorrichtung und ein Verfahren zur Ermittlung und Auswertung von Messdaten, die Körpereigenschaften, Körperfunktionen und, oder Vitalparametern eines Patienten umfassen können, aufweisend wenigstens eine Messwerteaufnahmevorrichtung zur Messung von Messdaten und wenigstens einer Auswertungs-, Anzeige- und, oder Bedieneinheit zur Auswertung und Weiterverarbeitung der Messdaten, wobei die Messwerteaufnahmevorrichtung und die Auswertungs-, Anzeige- und, oder Bedieneinheit des Vermessungssystems zueinander entkoppelt sind, wobei für die Kommunikation und, oder Datenübertragung zwischen der wenigstens einen Messwerteaufnahmevorrichtung und der Auswertungs-, Anzeige- und, oder Bedieneinheit eine Drahtlosübertragungseinrichtung vorgesehen und das Vermessungssystem derart ausgestaltet ist, dass Störeinflüsse auf die Messwerteaufnahme und, oder Messwerteergebnisse und, oder die Umwelt reduziert sind. Weiterhin umfasst die Erfindung ein Verfahren zur Ermittlung und Auswertung von Messdaten bestehend aus Körpereigenschaften, Körperfunktionen und, oder Vitalparametern eines Patienten.

## Beschreibung

Die Erfindung betrifft eine Vorrichtung zur Ermittlung und Auswertung von Messdaten, die Körpereigenschaften, Körperfunktionen und, oder Vitalparameter eines Patienten umfassen können, aufweisend wenigstens eine Messwerteaufnahmevorrichtung zur Messung von Messdaten und wenigstens einer Auswertungs-, Anzeige- und, oder Bedieneinheit zur Auswertung und Weiterverarbeitung der Messdaten, wobei die Messwerteaufnahmevorrichtung und die Auswertungs-, Anzeige- und, oder Bedieneinheit des Vermessungssystems zueinander entkoppelt sind.

Weiterhin umfasst die Erfindung ein Verfahren zur Ermittlung und Auswertung von Messdaten bestehend aus Körpereigenschaften, Körperfunktionen und, oder Vitalparametern eines Patienten.

Zur Messung von Körpereigenschaften und Körperfunktionen von Subjekten, z.B. menschlichen Personen oder Tieren, werden sowohl mobile als auch stationäre Messgeräte eingesetzt. Bei den zu messenden Eigenschaften oder Funktionen handelt es sich häufig um Bio- bzw. Körperimpedanzen, Herzfunktionen, Nervenfunktionen oder Elektrokardiogrammen. Weitere Messgrößen in diesem Zusammenhang können Körpergewicht, Körperzusammensetzungen, geometrische Abmessungen, Fingerabdrücke, Körpertemperaturen an verschiedenen Stellen, Zusammensetzung des verwerteten Atemgases, Atemgasvolumina, Blutdruckwerte usw. sein.

Für diese Messungen ist es erforderlich, dass das Messgerät mit dem zu messenden Subjekt verbunden wird und, oder dass die jeweiligen Messgrößen in geeigneter Weise gemessen oder erfasst und häufig auch verarbeitet und weitergeleitet werden. Ob eine Messung durch Kontakt mit dem Patienten oder kontaktlos erfolgen kann, ist abhängig von der Körpereigenschaft, der Körperfunktion bzw. dem Vitalparameter der gemessen werden soll und der dazu verwendeten Messmethode.

Die verfügbare Messtechnik generell und insbesondere in der Medizintechnik erlaubt heute, dass immer mehr Vitalparameter, verschiedene Körpereigenschaften und Körperfunktionen in immer kürzerer Zeit ermittelt und elektronisch weiterverarbeitet werden können.

Damit die Untersuchungskosten gering bleiben, sind wirtschaftlich optimierte Verarbeitungs- und Auswertungsprozesse der jeweiligen Messgrößen erforderlich. Um dieses Ziel zu erreichen, werden insbesondere in der Anwendung von Krankenhäusern und großen Gemeinschaftspraxen, in denen sehr viele Messwerte von einer Vielzahl von Patienten aufgenommen werden müssen, nicht mehr Vorrichtungskombinationen bestehend aus einer Messwerteaufnahmevorrichtung und einer zugeordneten Messwertverarbeitungs- und Auswertevorrichtung eingesetzt, sondern es werden durch eine Vielzahl von Messwerteaufnahmevorrichtungen die aufgenommenen Messwerte einer meist zentralen Messwertverarbeitungs- und Auswertevorrichtung zugeleitet.

Dieses Vorgehen ist eine Konsequenz aus der Tatsache, dass immer mehr Funktionalitäten von intelligenten Sensoren ohne komplizierte Anzeige- und Bedieneinheiten ausgeführt werden müssen und statt einer dezentralen Messwertverarbeitung durch einzelne Messgeräte vermehrt zentrale Auswertungs-, Anzeige- und Bedieneinheiten mit entsprechender Vernetzung eingesetzt werden. Dabei müssen die als Satelliten bezeichneten Messwerteaufnahmevorrichtungen eindeutig einem Patienten zuordbar sein.

Durch die Verwendung von physisch und häufig räumlich getrennten Messwerteaufnahmevorrichtungen wird auch eine andere, oftmals zu erfüllende Anforderung wenigstens teilweise realisiert, die in der Entkopplung der Messwerteaufnahmevorrichtungen und dem Messgerät, Auswertungs-, Anzeige- und, öder Bedieneinheit liegt. Die Entkopplung wird im Idealfall derart ausgebildet, dass eine galvanische Trennung vorliegt.

Die galvanische Trennung entkoppelt die Messwerteaufnahmevorrichtungen von dem Messgerät und, oder Auswertungs-, Anzeige- und, oder Bedieneinheiten in zweifacher Hinsicht - zum einen werden elektromagnetische Störungen wenigstens reduziert und zum anderen wird durch die galvanische Trennung eine Schutztrennung mit dem Ziel geschaffen, den Patienten vor elektrischen Schlägen bei Defekten des Messgerätes und, oder Auswertungs-, Anzeigeund, oder Bedieneinheit zu schützen.

Durch eine Entkopplung bzw. einer physischen Trennung der Messwerteaufnahmevorrichtungen von dem Messgerät und, oder Auswertungs-, Anzeigeund, oder Bedieneinheiten ist es erforderlich, dass die durch die Messwerteaufnahmevorrichtung ermittelten Messwerte der jeweiligen Körpereigenschaft, der Körperfunktion bzw. dem Vitalparameter an das Messgerät, Auswertungs-, Anzeige- und, oder Bedieneinheiten übertragen werden.

Generell kommen für Datenübertragungsaufgaben drahtgebundene und drahtlose Übertragungsprinzipe in Betracht, wobei die durch die Entkopplung gewünschte galvanische Trennung der Messwerteaufnahmevorrichtungen von dem Messgerät und, oder Auswertungs-, Anzeige- und, oder Bedieneinheiten nur mit Drahtlosprinzipen zur Datenübertragung realisierbar ist.

Drahtgebundene Übertragungsprinzipe sind einsetzbar bei entsprechend räumlichen Voraussetzungen, geringen Mobilitätsanforderungen an die Messwerteaufnahmevorrichtungen und, oder den Messgeräten bzw. den Auswertungs-, Anzeige- und, oder Bedieneinheiten. Soll eine vollständige galvanische Trennung realisiert werden, kommen drahtgebundene Übertragungsprinzipe nicht in Betracht

Die drahtgebundenen Übertragungsprinzipe zeichnen sich durch hohe Datenübertragungsgeschwindigkeiten und -volumina, kostengünstige Übertragungsvorrichtungen und hohe Übertragungssicherheit aus und verursachen bei entsprechender Ausgestaltung der beteiligten Komponenten zur Datenübertragung nur im direkten Umfeld und ohne erhebliche Reichweite elektromagnetische Emissionen.

Die drahtlosen Übertragungsprinzipe sind einsetzbar und vorteilhaft bei galvanischer Trennung und höheren Mobilitätsanforderungen an die Messwerteaufnahmevorrichtungen und, oder den Messgeräten bzw. den Auswertungs-, Anzeige- und, oder Bedieneinheiten.

Die drahtlosen Übertragungsprinzipe zeichnen sich durch praktisch uneingeschränkte Mobilität und Unabhängigkeit von räumlichen Ausstattungsvoraussetzungen wie Übertragungsleitungen, Kabelschächten und dergleichen aus.

Weiterhin ergeben sich erhebliche Vorteile praktischer Natur dadurch, dass die oft störenden Kabelverbindungen und Steckverbindungen beziehungsweise die Buchsen an den Geräten nicht benötigt werden.

Der Vorteil der uneingeschränkten Mobilität und Unabhängigkeit von räumlichen Ausstattungsvoraussetzungen sowie die Handhabungsvorteile können jedoch nur unter zwei weiteren Voraussetzungen maximal genutzt werden: Zum einen muss die ortsunabhängige Energieversorgung sichergestellt sein und zum anderen sind mögliche negative Auswirkungen durch die mit drahtlosen Übertragungsprinzipen für Daten und Energie häufig einhergehenden erhöhten elektromagnetischen Emissionen zu beachten.

Nur durch eine ortsunabhängige Energieversorgung insbesondere der Messwerteaufnahmevorrichtungen kann der Vorteil der Ortsunabhängigkeit durch die Verwendung drahtloser Datenübertragungssysteme vollständig genutzt werden.

Um die Energieversorgung von Energieverbrauchern generell ortsunabhängig zu realisieren, kommen zwei Vorgehensweisen in Betracht: Die Speicherung von Energie innerhalb des Energieverbrauchers oder eine drahtungebundene Übertragung, die ggf. auch eine Energiewandlung beinhaltet.

Bei der Energiespeicherung können Batterien, aufladbare Akkumulatoren oder andere speicherbare Energieträger, wie zum Beispiel Brennstoffe oder Reaktionssubstanzen, Verwendung finden.

Eine andere Möglichkeit zur ortsunabhängigen Versorgung mit Energie ist die drahtlose Zuführung. Dazu sind basierend auf verschiedenen Wirkprinzipen Lösungen entwickelt worden. Neben den kapazitiven, optischen und elektromagnetischen Verfahren kommt in der Medizintechnik vor alle die induktive Energieübertragung in Betracht. Eine andere als die induktive Übertragung kommt aus Kosten- und Effizienzgründen für die Medizintechnik nur für bestimmte Anwendungen in Frage.

Die drahtlose Energieübertragung kann innerhalb des Energieempfängers direkt zur Deckung des Energiebedarfs und, oder beispielsweise durch Aufladung einer Batterie gespeichert werden.

Die durch die drahtlose induktive Energieversorgung gewonnene örtliche Unabhängigkeit und die Handhabuhgsvorteile in der praktischen Anwendung bei der Vermessung von Patienten geht einher mit dem erheblichen Nachteil, dass erhöhte elektromagnetische Emissionen vorliegen. Ursächlich dafür ist das diesem Übertragungsprinzip zugrundeliegende, amplitudenmodulierte Hochfrequenzmagnetfeld zur Spannungs-induzierung.

Die erhöhten elektromagnetischen Emissionen durch die drahtlose Daten- oder Energieübertragungen können Wirkungen auf die zu vermessende Person oder das in der Nähe befindliches Bedienpersonal haben. Angrenzende Geräte und Einrichtungen, insbesondere empfindliche Instrumente in einer Intensivstation, können durch die elektromagnetischen Emissionen nachteilig beeinflusst oder beschädigt und in ihrer Funktion beeinträchtigt werden. Nicht zuletzt können auch die Messwerte verfälscht und damit die Ergebnisgüte reduziert werden.

Es ist daher Aufgabe der Erfindung ein Verfahren und eine Vorrichtung zur Vermessung von Patienten hinsichtlich seiner Körpereigenschaften, Körperfunktionen bzw. dessen Vitalparametern zu schaffen, bei dem eine Entkopplung und, oder physische Trennung der Messwerteaufnahmevorrichtungen von dem Messgerät oder einer Auswertungs-, Anzeige- und, oder Bedieneinheit realisiert und die die aufgezeigten Nachteile wenigstens teilweise überwindet oder reduziert.

Insbesondere ist es Aufgabe der Erfindung, die Handhabung möglichst einfach zu gestalten und Störeinflüsse auf die zu vermessende Person oder das Bedienpersonal, die im Umfeld befindliche Geräte und Einrichtungen und die Messwertergebnisse zu reduzieren.

Diese Aufgabenstellung löst die Erfindung durch ein Vermessungssystem aufweisend wenigstens eine Messwerteaufnahmevorrichtung zur Messung von Körpereigenschaften, Körperfunktionen und, oder Vitalparametern eines Patienten sowie einer von der Messwerteaufnahmevorrichtung entkoppelten Auswertungs-, Anzeige- und, oder Bedieneinheit, wobei wenigstens für die Kommunikation und, oder Datenübertragung zwischen der wenigstens einen Messwerteaufnahmevorrichtung und der Auswertungs-, Anzeige- und, oder Bedieneinheit ein Drahtlosverfahren vorgesehen und derart ausgestaltet ist, dass Störeinflüsse auf die Messwerteaufnahme und, oder Messwerteergebnisse und, oder die Umwelt reduziert sind

Die erfindungsgemäße Lehre erkennt, dass die Händhabungs- und, oder Mobilitätsverbesserung durch ein Drahtloskommunikations- und, oder Datenübertragungsprinzip zwischen den entkoppelten Elementen eines Vermessungssystems nur unter der Voraussetzung vorteilhaft nutzbar sind, dass mit der Drahtlosübertragungstechnik die Messwertergebnisse in ihrer Ergebnisgüte nicht negativ beeinflusst werden und schlägt eine Reihe von Maßnahmen vor. Wird zusätzlich zur drahtlosen Kommunikation und, oder Datenübertragung auch die Energieversorgung drahtlos realisiert, ist auch diese von den Maßnahmen zur Verringerung der Störeinflüsse auf die Messwertergebnisse umfasst.

Eine erste Ausgestaltung der erfindungsgemäßen Vermessungssystems mit zueinander entkoppelten Elementen in Form von wenigstens einer Messwerteaufnahmevorrichtung und einer Auswertungs-, Anzeige- und, oder Bedieneinheit bzw. einer Zentraleinheit sieht vor, dass während eines Messvorganges von Körpereigenschaften, Körperfunktionen und, oder Vitalparametern eines Patienten durch die Messwerteaufnahmevorrichtung keine simultane Kommunikation und, oder Datenübertragung stattfindet.

Vielmehr sieht die Erfindung eine zeitversetzte, d.h. der Messung nachgelagerte Kommunikation und, oder Datenübertragung von der Messwerteaufnahmevorrichtung zu der Auswertungs-, Anzeige- und, oder Bedieneinheit vor. Gerätetechnisch wird dazu ein geeignetes Speichermedium zur wenigstens temporären Speicherung von Mess- und, oder Kommunikationsinformationen in die Messwerteaufnahmevorrichtung integriert.

Die erfindungsgemäße Lehre erkennt weiterhin, dass Störeinflüsse auf die Ergebnisgüte der Messwertergebnisse durch eine möglichst geringe Sendeleistung der Drahtlosübertragungsprinzipe basierend auf der Aussendung von energetischen Wellen, beispielsweise Funkwellen oder WLAN-basierte Übertragung, reduzierbar sind.

Die Vermeidung von Kabeln und Steckern unterstützt die einfache Durchführung von Reinigungsvorgängen sowie eine einfache Handhabung. Es wird insbesondere durch eine Vielzahl unterschiedlicher Stecker vermieden, dass eine falsche Konfiguration erfolgen kann und dass falsche Stecker und Buchsen miteinander kombiniert werden. Zusätzlich zu der Tatsache, dass infolge der Drahtlosübertragung an den Elementen des Vermessungssystems die Handhabung durch Reduzierung oder vollständigem Verzicht auf Steckverbindungen, Buchsen, Anschlusskoppelungen und dergleichen verbessert wird, erkennt die Erfindung auch, dass der wenigstens weitgehende Verzicht auf Steckverbindungen auch die Störeinflüsse auf die Messergebnisse reduziert. Auch bei einer drahtgebundenen Datenübertragung und, oder Stromversorgung werden, insbesondere an den Steckverbindungen und Buchsen, elektromagnetische Emissionen erzeugt.

Gemäß einer weiteren Ausführungsform des Vermessungssystems ist eine Kapselung wenigstens der elektromagnetischen Emittenten vorgesehen. Hierdurch wird die Umwelt generell und damit auch der Patient oder andere Einrichtungen und Geräte vor bestimmten äußeren Einwirkungen geschützt. Die Störeinflüsse auf Messwerte bei deren Aufnahme oder Übertragung wird reduziert.

Um die Störeinflüsse durch elektromagnetische Strahlung weiter zu reduzieren, sieht die Erfindung im Rahmen einer weiteren Ausführungsform die Verwendung einer optischen Schnittstelle und damit das Infrarot- Drahtlosübertragungsprinzip vor. Mit diesem Prinzip wird die Ausbildung unerwünschter elektromagnetischer Felder reduziert und die elektromagnetische Verträglichkeit unterstützt. Die ansonsten durch die Verwendung von Antennen hervorgerufene Streustrahlung wird vermieden.

Neben der Energieversorgung der wenigstens einen Messwerteaufnahmevorrichtung und der Auswertungs-, Anzeige- und, oder Bedieneinheit als entkoppelte Elemente des Vermessungssystems durch Batterien oder wiederaufladbaren Akkumulatoren sieht die Erfindung auch die Verwendung drahtlos arbeitender Energieübertragungssysteme vor. Insbesondere die induktive Energieübertragung in Verbindung bzw. dem Ziel einer vollständigen galvanischen Trennung der Vermessungssystemelemente ist vorgesehen. Die induktive DrahtlosEnergieübertragung ist, wie die Drahtlos-Übertragungprinzipe basierend auf energiereichen Wellen auch, ein elektromagnetischer Emittent.

Gemäß einer weiteren bevorzugten Ausführungsform wird die Entkopplung der Vermessungssystemelemente, d.h. der wenigstens einen Messwerteaufnahmevorrichtung und der Auswertungs-, Anzeige- und, oder Bedieneinheit durch eine räumliche Trennung unterstützt.

Um die Messwertegenauigkeit in Anbetracht der nicht vollständig auszuschließenden Störeinflüsse weiter zu maximieren beinhaltet die erfindungsgemäße Lehre weiterhin Maßnahmen zur Fehlerkorrektur, die auf die verbleibenden Reststörungen abgestimmt sind. Auftretende Spiegelfrequenzen können durch geeignete Kombination von Analog- und Digitalfiltern reduziert werden. Referenzmesswerte, die in Abwesenheit von elektromagnetischen Emissionen ermittelt werden können als Skalierungsmaßstab und, oder für Plausibilitätschecks herangezogen werden. Sind Abweichungen ausserhalb einer zuvor festgelegten Toleranzschwelle zu verzeichnen, kann eine umgehende Wiederholungsmessung initiiert werden.

Die Erfindung wird nachfolgend mit Bezug auf die Figuren erläutert. Im Einzelnen zeigen
- Fig. 1: das schematische Beispiel des erfindungsgemäßen Vermessungssystems (1) mit Messwerteaufnahmevorrichtung (20) und einer Auswertungs-, Anzeige- und, oder Bedieneinheit (10),
- Fig. 2: das Ausführungsbeispiel des Vermessungssystems (1) mit durch räumliche Trennung entkoppelten Elementen (10, 20),
- Fig. 3: die Messwerteaufnahmevorrichtung (20) als ein Element des Vermessungssystems (1) bestehend im Wesentlichen aus einer Messmatte (21) und Messwerteaufnahmesensoren (22) sowie wenigstens einer schematisch angedeuteten Drahtlosübertragungseinrichtung (30),
- Fig. 4: illustriert ein Ausführungsbeispiel der Messwerteaufnahmevorrichtung (20) ausgebildet basierend auf einer Messmatte (21) in seiner Anwendung bei der Messung an einem Patienten.

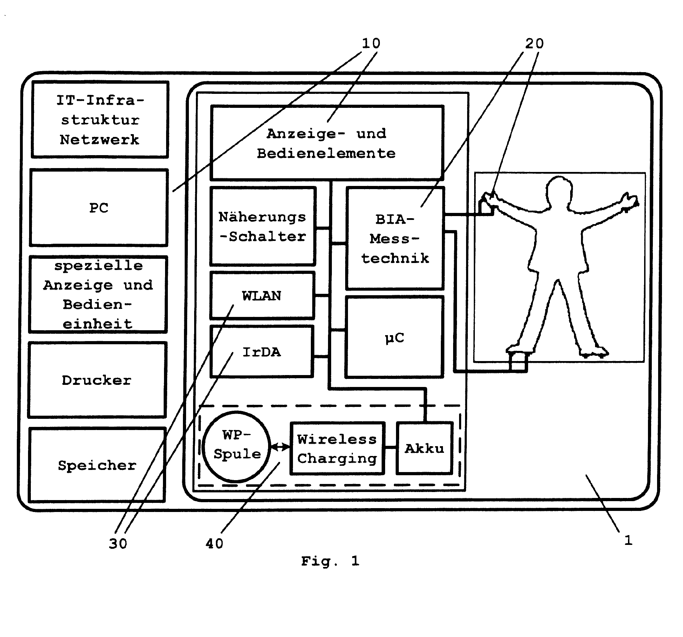

Figur 1 zeit das schematische Beispiel des erfindungsgemäßen Vermessungssystems (1) mit Messwerteaufnahmevorrichtung (20) und einer davon entkoppelten Auswertungs-, Anzeige- und, oder Bedieneinheit (10), wobei die Elemente (10, 20) des Vermessungssystems (1) sowohl räumlich getrennt als auch ohne räumliche Trennung, aber entkoppelt ausgeführt sein können. Damit die Elemente (10, 20) zueinander kommunizieren und, oder Daten übertragen können, sind jeweils drahtgebundene oder drahtlos arbeitende Übertragungseinrichtungen vorgesehen. Bei der Verwendung von Drahtlosübertragungseinrichtungen (30) können verschiedene Wirkprinzipe eingesetzt werden, beispielsweise optische Verfahren in Form von Infrarot oder funkbasierende Verfahren wie WLAN oder Bluetooth.

Schematisch und beispielhaft ist in Figur 1 auch die erforderliche Energieversorgung (40) der Elemente (10, 20) des Vermessungssystems (1) dargestellt. Je nach realisierter Entkopplung kann eine gemeinsame oder für jedes Element (10, 20) separate Stromversorgung vorgesehen sein, die auf drahtgebundenen oder drahtlosen Verfahren beruht. Skizziert ist in Figur 1 eine für beide Elemente gemeinsam arbeitende drahtlose Versorgung dergestalt, dass per Induktionsspule ein Akkumulator drahtlos geladen wird, der dann die Stromversorgung bereitstellt.

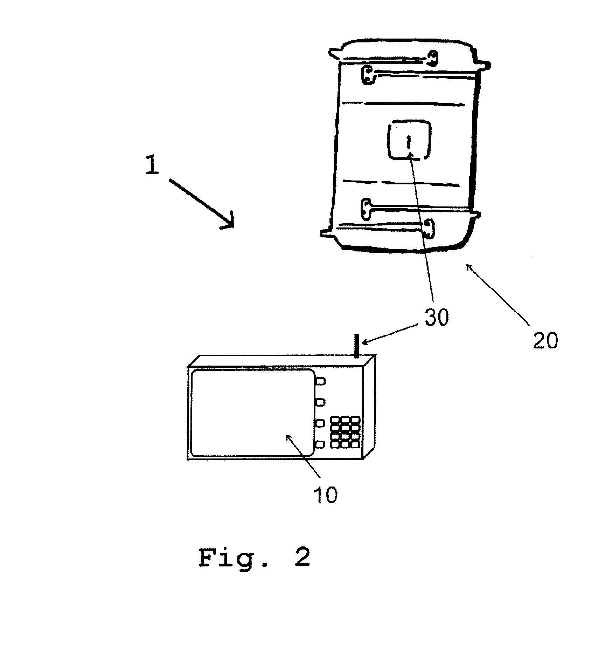

Figur 2 bildet das Ausführungsbeispiel des Vermessungssystems (1) mit durch räumliche Trennung entkoppelten Elementen (10, 20) ab. Dieses Ausführungsbeispiel umfasst wenigstens für die Kommunikations- und Datenübertragungsfunktion zwischen der wenigstens einen Messwerteaufnahmevorrichtung (20) und der Auswertungs-, Anzeige- und, oder Bedieneinheit (10), die auch als Zentraleinheit ausgebildet sein kann, ein Drahtlosübertragungsprinzip.

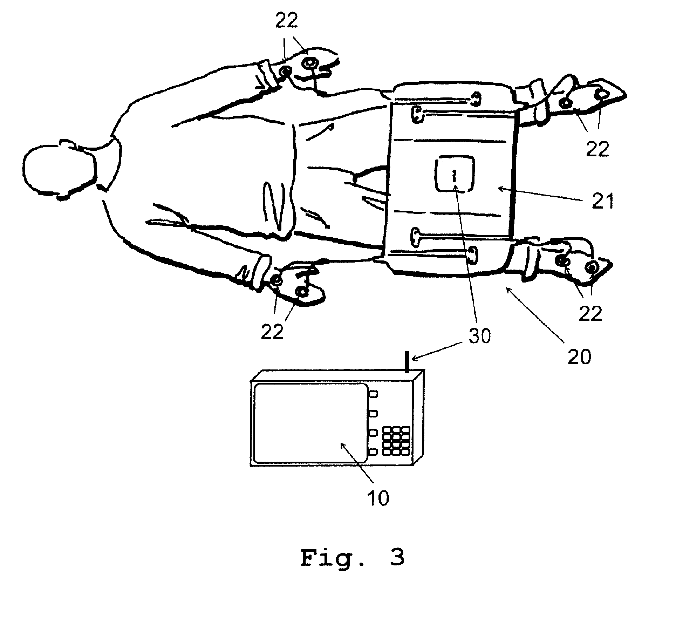

Figur 3 zeigt die Messwerteaufnahmevorrichtung (20) als ein Element des Vermessungssystems (1), bestehend im Wesentlichen aus einer Messmatte (21) und Messwerteaufnahmesensoren (22) sowie wenigstens einer schematisch angedeuteten Drahtlosübertragungseinrichtung (30) für die Kommunikation und, oder den Datenaustausch mit der Auswertungs-, Anzeige- und, oder Bedieneinheit (10). Die erforderliche Strom- bzw. Energieversorgung ist in Figur 3 nicht dargestellt, kann aber sowohl drahtgebunden als auch drahtlos erfolgen.

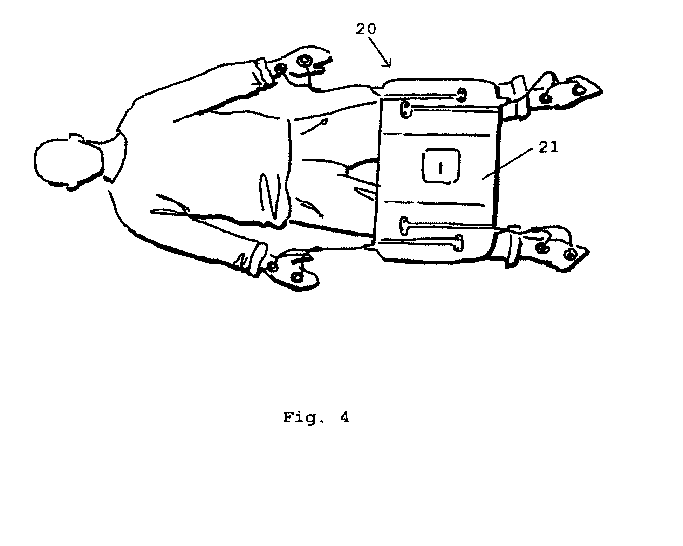

Figur 4 illustriert ein Ausführungsbeispiel der Messwerteaufnahmevorrichtung (20) ausgebildet basierend auf einer Messmatte (21) in seiner Anwendung bei der Messung an einem Patienten. Die Ausgestaltung der Messwerteaufnahmevorrichtung (20) basierend auf einer Messmatte (21) ist für das erfindungsgemäße Vermessungssystem (1) besonders vorteilhaft, da die Messwertsensoren (22) sowie Energieversorgung (40) und die ggf. erforderliche Drahtlosübertragungseinrichtung (30) unproblematisch in die Messmatte (21) integrierbar sind.

Gemäß einer weiteren Variante der Erfindung ist auch daran gedacht, dass alternativ oder ergänzend zu drahtgebundenen oder nicht drahtgebundenen Verbindungen Glasfaserverbindungen (GOF, POF) realisierbar sind, wobei diese die Emissionen vermeiden, aber trotzdem eine galvanische Trennung realisieren.

Bei einer drahtlosen Energieübertragung ist insbesondere daran gedacht, nur dann zu laden, wenn die Matte im Monitor hängt. Die WLAN-Übertragung kann jedoch bei der Messung aktiv sein.

Insbesondere ist vorgesehen, dass die Auswertungs-, Anzeige- und, oder Bedieneinheit (10) des Vermessungssystems (1) eine Zentraleinheit ist.

Insbesondere ist vorgesehen, dass die Schritte a.) und b.) zeitgleich erfolgen.

Insbesondere ist vorgesehen, dass die Schritte a.) und b.) zeitversetzt zueinander erfolgen.

Insbesondere ist vorgesehen, dass nach Schritt a.) und, oder b.) Fehlerkorrekturmaßnahmen durchgeführt werden, die auf die verbleibenden Reststöreinflüsse abgestimmt sind.

Insbesondere ist vorgesehen, dass die Fehlerkorrekturmaßnahmen aus Analogund Digitalfilterungen bestehen, sodass Spiegelfrequenzen reduziert werden.

Insbesondere ist vorgesehen, dass die Fehlerkorrekturmaßnahmen aus Skalierungskorrekturen anhand von Referenzmesswerten ermittelt in Abwesenheit von elektromagnetischen Emissionen bestehen, sodass Messwerteveränderungen durch elektromagnetische Störeinflüsse reduziert werden.

Insbesondere ist vorgesehen, dass die Fehlerkorrekturmaßnahmen aus Plausibilitätschecks anhand von Referenzmesswerten ermittelt in Abwesenheit von elektromagnetischen Emissionen bestehen, sodass Messwerteveränderungen durch elektromagnetische Störeinflüsse erkannt und Wiederholungsmessungen initiiert werden.

## Patentansprüche

1. Vorrichtung (1) zur Ermittlung und Auswertung von Messdaten, die Körpereigenschaften, Körperfunktionen und, oder Vitalparametern eines Patienten umfassen können, aufweisend wenigstens eine Messwerteaufnahmevorrichtung (20) zur Messung von Messdaten und wenigstens einer Auswertungs-, Anzeige- und, oder Bedieneinheit (10) zur Auswertung und Weiterverarbeitung der Messdaten, wobei die Messwerteaufnahmevorrichtung (20) und die Auswertungs-, Anzeige- und, oder Bedieneinheit (10) des Vermessungssystems (1) zueinander entkoppelt sind, **dadurch gekennzeichnet, dass** für die Kommunikation und, oder Datenübertragung zwischen der wenigstens einen Messwerteaufnahmevorrichtung (20) und der Auswertungs-, Anzeige- und, oder Bedieneinheit (10) eine Drahtlosübertragungseinrichtung (30) vorgesehen und das Vermessungssystem (1) derart ausgestaltet ist, dass Störeinflüsse auf die Messwerteaufnahme und, oder Messwerteergebnisse und, oder die Umwelt reduziert sind.

2. Vorrichtung (1) nach Anspruch 1, **dadurch gekennzeichnet, dass** die Messwerteaufnahmevorrichtung (20) und die Auswertungs-, Anzeige- und, oder Bedieneinheit (10) des Vermessungssystems (1) zueinander durch eine vollständige galvanischen Trennung entkoppelt sind.

3. Vorrichtung (1) nach Anspruch 1, **dadurch gekennzeichnet, dass** die Messwerteaufnahmevorrichtung (20) und die Auswertungs-, Anzeige- und, oder Bedieneinheit (10) des Vermessungssystems (1) zueinander durch eine räumliche Trennung die Entkopplung unterstützt.

4. Vorrichtung (1) nach Anspruch 1, **dadurch gekennzeichnet, dass** die Drahtlosübertragungseinrichtung (30) durch eine NFC- oder Infrarot- oder WLAN- oder Bluetooth-Schnittstelle gebildet ist.

5. Vorrichtung (1) nach Anspruch 1, **dadurch gekennzeichnet, dass** die Sendeleistung der Drahtlosübertragungseinrichtung (30) auf das das Mindestniveau zur Übertragung von Daten und, oder zur Kommunikation angepasst ist, sodass Störeinflüsse auf die Messwerteaufnahme und, oder Messwerteergebnisse und, oder die Umwelt reduziert sind.

6. Vorrichtung (1) nach Anspruch 1, **dadurch gekennzeichnet, dass** die wenigstens eine Messwerteaufnahmevorrichtung (20) wenigstens eine Datenspeichervorrichtung zur temporären oder dauerhaften Speicherung von Messwerten aufweist, sodass ein zur Messwerteaufnahme zeitversetzter Datentransfer unterstützt ist.

7. Vorrichtung (1) nach Anspruch 1, **dadurch gekennzeichnet, dass** wenigstens ein Element (10, 20) des Vermessungssystems (1) durch Batterien oder wiederaufladbare Akkumulatoren energieversorgt ist.

8. Vorrichtung (1) nach Anspruch 1, **dadurch gekennzeichnet, dass** wenigstens ein Element (10, 20) des Vermessungssystems (1) durch ein drahtlos arbeitendes Energieübertragungssystem energieversorgt ist.

9. Vorrichtung (1) nach Anspruch 8, **dadurch gekennzeichnet, dass** das drahtlos arbeitende Energieübertragungssystem die Entkopplung der Elemente (10, 20) unterstützt.

10. Vorrichtung (1) nach Anspruch 8, **dadurch gekennzeichnet, dass** das drahtlos arbeitende Energieübertragungssystem induktiv arbeitet, sodass eine Stromversorgung zur direkten Bedarfsdeckung des Elementes (10, 20) oder zur Aufladung eines Akkumulators erfolgt.

11. Vorrichtung (1) nach Anspruch 1, **dadurch gekennzeichnet, dass** wenigstens ein elektromagnetischer Emittent des Vermessungssystems (1) gekapselt ist, sodass Störeinflüsse auf die Messwerteaufnahme und, oder Messwerteergebnisse und, oder die Umwelt reduziert sind.

12. Vorrichtung (1) nach Anspruch 1, **dadurch gekennzeichnet, dass** wenigstens ein Element (10, 20) des Vermessungssystems (1) gekapselt ist, sodass Störeinflüsse auf die Messwerteaufnahme und, oder Messwerteergebnisse und, oder die Umwelt reduziert sind.

13. Vorrichtung (1) nach Anspruch 1, **dadurch gekennzeichnet, dass** die Messwerteaufnahmevorrichtung (20) eine Messmatte (21) und Messwerteaufnahmesensoren (22) sowie wenigstens eine Drahtlosübertragungseinrichtung (30) und eine Energieversorgung (40) aufweist.

14. Vorrichtung (1) nach Anspruch 13, **dadurch gekennzeichnet, dass** die wenigstens eine Drahtlosübertragungseinrichtung (30) und die Energieversorgung (40) in die Messmatte (21) integriert sind.

15. Verfahren zur Ermittlung und Auswertung von Körpereigenschaften, Körperfunktionen und, oder Vitalparametern eines Patienten, aufweisend die Schritte
a.) Messen der Körpereigenschaften, Körperfunktionen und, oder Vitalparameter,
b.) drahtlose Übertragung der gemessenen und erfassten Daten an die Auswertungs-, Anzeige- und, oder Bedieneinheit (10) des Vermessungssystems (1), **dadurch gekennzeichnet, dass** die gemessenen und, oder erfassten Daten derart drahtlos übertragen werden, sodass Störeinflüsse auf die Messwerteaufnahme und, öder Messwerteergebnisse und, oder die Umwelt reduziert sind.
